(19) Europäisches Patentamt
European Patent Office
Office européen des brevets

(11) **EP 1 061 367 A2**

(12) **EUROPÄISCHE PATENTANMELDUNG**

(43) Veröffentlichungstag:
20.12.2000 Patentblatt 2000/51

(51) Int. Cl.[7]: **G01N 33/36**

(21) Anmeldenummer: **00111374.5**

(22) Anmeldetag: **26.05.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **29.05.1999 DE 19924840**

(71) Anmelder: **Röhm, Klaus**
**72764 Reutlingen (DE)**

(72) Erfinder: **Röhm, Klaus**
**72764 Reutlingen (DE)**

(74) Vertreter:
**Möbus, Daniela, Dr.-Ing.**
**Patentanwälte Dipl.-Ing. Rudolf Möbus,**
**Dr.-Ing. Daniela Möbus,**
**Dipl.-Ing. Gerhard Schwan,**
**Hindenburgstrasse 65**
**72762 Reutlingen (DE)**

(54) **Vorrichtung zur Bestimmung der Haarigkeit von textilen Gebilden**

(57)    Eine Vorrichtung zur Bestimmung der Haarigkeit von textilen Gebilden mit mindestens einem die von der Oberfläche des textilen Gebildes reflektierten Lichtstrahl sensierenden optischen Detektor sowie einer Auswerteeinrichtung für die Signale des mindestens einen Detektors, wobei die Auswerteeinrichtung einen Haarigkeitskennwert $H_q$ unter Berücksichtigung einer relativen Haarlängenklassenhäufigkeit $R_k$ und des Moments M der Häufigkeitsverteilung bestimmt.

Definition der Haarigkeitsmerkmale — Fig. 1

**Beschreibung**

Die bisher benutzten Messeinrichtungen für die Haarigkeitsmessung an Textilien verwenden Zeilen- bzw. Flächenkameras oder Einzeldioden im Zusammenhang mit einer Dunkelfeld- bzw. Hellfeldbeleuchtung des Messobjekts. Die Messsignale werden vornehmlich nach Schwellwertverfahren ausgewertet. Diese Methode ist relativ grob und ermöglicht somit keine sehr differenzierte Unterscheidung der Haarigkeit verschiedener textiler Gebilde. Bei bekannten Messgeräten, wie z. B. das Gerät E 566 der Firma Zweigle, werden histogrammartige Balkendarstellungen zur Ergebnisvisualisierung und Auswertung benutzt. Dabei werden physikalisch angenähert die tatsächliche Haarlänge und die Faserzahl pro Messpunkt ermittelt. Aus diesen Darstellungen lässt sich optisch relativ einfach die Abweichung der Haarigkeit eines untersuchten Objekts von der erwarteten theoretischen Haarigkeit entsprechend dem Material und der Verarbeitung des textilen Gebildes feststellen.

Der vorliegenden Erfindung liegt die Aufgabe zugrunde, eine Vorrichtung vorzuschlagen, mit der eine differenzierte Unterscheidung der Haarigkeit verschiedener textiler Gebilde möglich ist.

Die Aufgabe wird durch eine Vorrichtung mit den Merkmalen des Patentanspruchs 1 gelöst. Anspruch 2 gibt eine bevorzugte Ausgestaltung der Vorrichtung an.

Mit der erfindungsgemäßen Vorrichtung wird nicht nur die maximale Haarlänge, sondern auch die dazwischenliegenden Haarlängen und damit eine so genannte Haarigkeitsdichteverteilung ermittelt. Dadurch lassen sich wesentlich differenziertere Aussagen über die Haarigkeit eines textilen Gebildes machen. Die Haarigkeitsdichteverteilung kann aus einem Haarlängenmaximalwert bzw. aus mehreren Haarlängenmesswerten an einer Messstelle des Objekts bestehen. Die Haarigkeitsdichteverteilung kann theoretisch auch als angenäherte physikalische Massenverteilung der abstehenden Haare des textilen Gebildes angesehen werden, wenn man voraussetzt, dass die Materialdichte des textilen Gebildes ausreichend konstant ist und die durch das Messsystem projizierte Fläche sozusagen einer definierten Messfläche entspricht. Dadurch ist eine mögliche Korrelation der Massenverteilung an z. B. Garnen denkbar und mit der erfindungsgemäßen Vorrichtung ermittelbar.

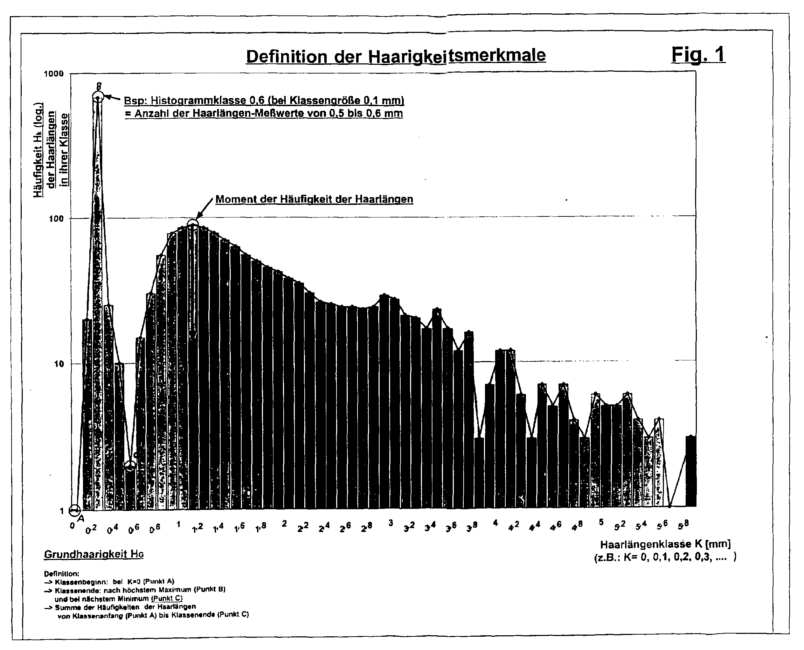

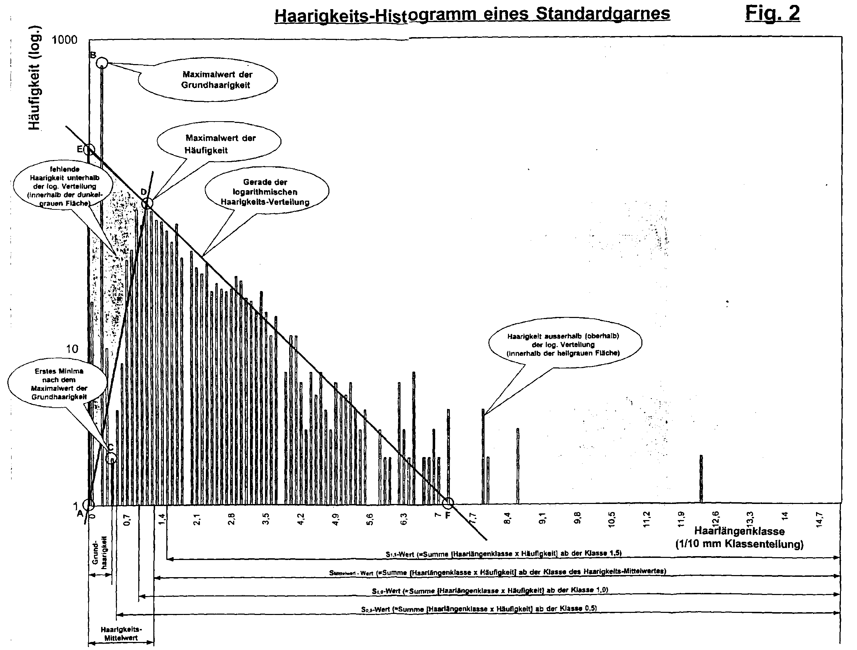

Weitere wichtige Haarigkeitsmerkmale werden anhand der beiliegenden Tabelle sowie der Fig. 1 und 2 angegeben. Alle diese Merkmale können ebenfalls von der Auswerteeinrichtung bestimmt werden.

Insbesondere durch die Kombination mehrerer Haarigkeitsmerkmale lässt sich eine sehr differenzierte Qualitätsunterscheidung von textilen Gebilden durchführen. Die Vorrichtung eignet sich beispielsweise zur Kontrolle der produzierten Garnqualität bzw. zur Beeinflussung der Garnqualitäten wie Weiterverarbeitungseigenschaften, Haarigkeit, Festigkeit durch Korrelation und anderes, am Ende des Produktionsgangs einer Spinnmaschine oder anderer Textilmaschinen. Eine oder mehrere vorgegebene Entscheidungsschwellen bezüglich der Qualität ermöglichen das Sortieren der Textilien in der Textilmaschine oder am Ende der Textilmaschine mittels einer dadurch angesteuerten Vorrichtung zur Sortierung der verschiedenen Qualitätsstufen bzw. von Ausschussprodukten. Eine Qualitätsbeeinflussung der Textilien aufgrund der Messergebnisse ist durch eine Rückkopplung zur Textilmaschine ebenfalls möglich. Das Messsystem wird dann in den Steuerungs- oder Regelkreis der Textilmaschine integriert.

Die Vorrichtung lässt sich insbesondere zur Haarigkeitsmessung der Kopse oder des Garns bei Spinnmaschinen aller Art einsetzen. Auch bei Spinnmaschinen ist sie zur Kontrolle der Kopse, des Garns und der Wickel einsetzbar. Bei garnverarbeitenden Maschinen wie Webmaschinen oder Strickmaschinen können mit der erfindungsgmäßen Vorrichtung das Garn, die Wickel sowie die erzeugten Flächengebilde untersucht werden. Das Gleiche gilt für Textilveredelungseinrichtungen wie Einrichtungen zum Sengen, bei denen mit der Vorrichtung Kopse, Wickel, Flächengebilde oder das Garn selbst untersucht werden können. Selbstverständlich ist ein Einsatz der Vorrichtung auch als Labormessgerät oder als tragbares Messgerät für die Qualitätskontrolle in anderen Bereichen zur Erfassung der Textilqualität möglich.

Tabelle 1.1

| Pkt. | Merkmal | Beschreibung |
|------|---------|--------------|
| 1 | Grundhaarigkeit $H_G$ des Haarigkeits-Maximal-Meßwertes | Siehe Definition in Fig. 1 in Verbindung der Meßwerte aus den Haarigkeits-Maximal-Meßwerten |
| 2 | Grundhaarigkeit $H_G$ der Haarigkeits-Dichteverteilung | Siehe Definition in Fig. 1 in Verbindung der Meßwerte aus den Haarigkeits-Dichteverteilungs-Meßwerten |
| 3 | Verhältnis der Meßwerte von Pkt. 1 und der Meßwerte zu Pkt 2 ausgedrückt in % | Verhältnis in % von Grundhaarigkeit $H_G$ des Haarigkeits-Maximal-Meßwertes zu Grundhaarigkeit $H_G$ der Haarigkeits-Dichteverteilung |

Tabelle 1.1 (fortgesetzt)

| Pkt. | Merkmal | Beschreibung |
|---|---|---|
| 4 | Differenz der Meßwerte von Pkt. 2 und Pkt. 1 | Differenz von Grundhaarigkeit $H_G$ der Haarigkeits-Dichteverteilung und der Grundhaarigkeit $H_G$ des Haarigkeits-Maximal-Meßwertes |
| 5 | Berechnete Haarigkeitsqualität $H_Q$ des Haarigkeits-Maximal-Meßwertes | Die Haarigkeitsqualität $H_Q$ berechnet sich nach den Gleichungen wie sie in Anspruch 2 beschrieben sind. |
| 6 | Berechnete Haarigkeitsqualität $H_Q$ der Meßwerte der Haarigkeits-Dichteverteilung | Die Haarigkeitsqualität $H_Q$ berechnet sich nach den Gleichungen wie sie in Anspruch 2 beschrieben sind. |
| 7 | Verhältnis der Meßwerte von Pkt. 5 und der Meßwerte zu Pkt 6 ausgedrückt in % | Verhältnis in % von Haarigkeitsqualität $H_Q$ des Haarigkeits-Maximal-Meßwertes zu Haarigkeitsqualität $H_Q$ der Haarigkeits-Dichteverteilung |
| 8 | Differenz der Meßwerte von Pkt. 5 und Pkt. 6 | Differenz von Haarigkeitsqualität $H_Q$ der Haarigkeits-Dichteverteilung und Haarigkeitsqualität $H_Q$ des Haarigkeits-Maximal-Meßwertes |
| 9 | Verhältnis der Meßwerte von Pkt. 1 und der Meßwerte zu Pkt 5 ausgedrückt in % | Verhältnis in % von Grundhaarigkeit $H_G$ des Haarigkeits-Maximal-Meßwertes zu Haarigkeitsqualität $H_Q$ des Haarigkeits-Maximal-Meßwertes |
| 10 | Verhältnis der Meßwerte von Pkt. 2 und der Meßwerte zu Pkt 6 ausgedrückt in % | Verhältnis in % von Grundhaarigkeit $H_G$ der Haarigkeits-Dichteverteilung zu Haarigkeitsqualität $H_Q$ der Haarigkeits-Dichteverteilung |

Tabelle 1.2

| Pkt. | Merkmal | Beschreibung |
|---|---|---|
| 11 | Haarigkeits-Meßwerte außerhalb der logarithmischen Verteilung (> der theoretischen Verteilung) | Siehe hierzu Fig. 2. Berechnete Werte z.B. ermittelt als Fläche (Haarlänge x Häufigkeit), als Moment der Häufigkeit der Haarlängen (Haarigkeitsqualität $H_Q$), Grundhaarigkeit $H_G$ aus den Haarigkeits-Maximal-Meßwerten und den Meßwerten der Haarigkeits-Dichteverteilung |
| 12 | Differenz der Haarigkeits-Meßwerte der theoretischen logarithmischen Verteilung und der tatsächlichen Verteilung (fehlende Haarigkeit unterhalb der log. theoretischen Verteilung) | Siehe hierzu Fig. 1. (schraffierte Fläche) Berechnete Werte z.B. ermittelt als Fläche (Haarlänge x Häufigkeit), als Moment der Häufigkeit der Haarlängen (Haarigkeitsqualität $H_Q$), Grundhaarigkeit $H_G$ aus den Haarigkeits-Maximal-Meßwerten und den Meßwerten der Haarigkeits-Dichteverteilung |
| 13 | Maximalwert Grundhaarigkeit | Siehe hierzu Fig. 1 + 2. Entspricht dem ersten höchsten Maximum (mit darauffolgendem deutlichem Minimum ) der Häufigkeitsverteilung (Häufigkeit der Klasse beim Maximum) aus den Haarigkeits-Maximal-Meßwerten und den Meßwerten der Haarigkeits-Dichteverteilung |

Tabelle 1.2 (fortgesetzt)

| Pkt. | Merkmal | Beschreibung |
|---|---|---|
| 14 | S-Mittelwert | Siehe hierzu Fig. 2.<br><br>Entspricht der Summe der Häufigkeiten ab dem bereits vorher ermittelten Haarigkeitsmittelwertes (Haarigkeit oberhalb des Mittelwertes) Berechnete Werte z.B. ermittelt als Fläche (Haarlänge x Häufigkeit), als Moment der Häufigkeit der Haarlängen (Haarigkeitsqualität $H_Q$), Grundhaarigkeit $H_G$ aus den Haarigkeits-Maximal-Meßwerten und den Meßwerten der Haarigkeits-Dichteverteilung |
| 15 | Steigung der Geraden der Grundhaarigkeit | Siehe hierzu Fig. 2.<br><br>Bestimmung der Steigung mittels z.B. Regressionsanalyse und Log-Funktionen oder anhand von anderen mathematischen Näherungsverfahren ausgehend vom Nullpunkt bzw. ersten Minima und dem zweiten Maxima der Häufigkeitsverteilung $H_G$ aus den Haarigkeits-Maximal-Meßwerten und den Meßwerten der Haarigkeits-Dichteverteilung |
| 16 | Steigung der Geraden der Log.-normalverteilten Haarigkeit (im Diagramm) | Siehe hierzu Fig. 2.<br><br>Bestimmung der Steigung mittels z.B. Regressionsanalyse und Log-Funktionen oder anhand von anderen mathematischen Näherungsverfahren $H_G$ aus den Haarigkeits-Maximal-Meßwerten und den Meßwerten der Haarigkeits-Dichteverteilung |

**Patentansprüche**

1. Vorrichtung zur Bestimmung der Haarigkeit von textilen Gebilden mit mindestens einer Beleuchtungseinrichtung und mindestens einem die von der Oberfläche des textilen Gebildes reflektierten Lichtstrahlen sensierenden optischen Detektors sowie einer Auswerteeinrichtung für die Signale des mindestens einen Detektors, dadurch gekennzeichnet, dass die Auswerteeinrichtung einen Haarigkeitskennwert $H_q$ unter Berücksichtigung einer relativen Haarlängenklassenhäufigkeit $R_k$ und des Moments M der Häufigkeitsverteilung bestimmt.

2. Vorrichtung nach Anspruch 1, dadurch gekennzeichnet, dass die Auswerteeinrichtung einen Haarigkeitskennwert $H_q$ nach der Beziehung

$$H_q = S \cdot \sum_{k=0}^{n} (k-M)^m \cdot R_k$$

ermittelt, wobei S ein Skalierungsfaktor, n die Anzahl der Haarlängenklassen des textilen Gebildes, m = 1, 2 oder 3 und $R_k$ eine relative Haarlängenklassenhäufigkeit nach der Beziehung

$$R_k = \frac{H_k}{\sum_{k=0}^{n} H_k}$$

ist, wobei $H_k$ die Häufigkeit der Messwerte in der Haarlängenklasse k ist und M das Moment der Häufigkeitsverteilung nach der Beziehung

$$M = \sum_{k=0}^{n} H_k \cdot R_k$$

ist.

# Definition der Haarigkeitsmerkmale

## Fig. 1

Bsp: Histogrammklasse 0,6 (bei Klassengröße 0,1 mm)
= Anzahl der Haarlängen-Meßwerte von 0,5 bis 0,6 mm

Moment der Häufigkeit der Haarlängen

Häufigkeit $H_k$ (log.) der Haarlängen in ihrer Klasse

Haarlängenklasse K [mm]
(z.B.: K= 0, 0,1, 0,2, 0,3, .... )

Grundhaarigkeit $H_G$

Definition:
→ Klassenbeginn: bei K=0 (Punkt A)
→ Klassenende: nach höchstem Maximum (Punkt B)
   und bei nächstem Minimum (Punkt C)
→ Summe der Häufigkeiten der Haarlängen
   von Klassenanfang (Punkt A) bis Klassenende (Punkt C)

EP 1 061 367 A2

EP 1 061 367 A2

Haarigkeits-Histogramm eines Standardgarnes    Fig. 2

Häufigkeit (log.)

Haarlängenklasse (1/10 mm Klassenteilung)

Maximalwert der Grundhaarigkeit

Maximalwert der Häufigkeit

Gerade der logarithmischen Haarigkeits-Verteilung

fehlende Haarigkeit unterhalb der log. Verteilung (innerhalb der dunkelgrauen Fläche)

Erstes Minima nach dem Maximalwert der Grundhaarigkeit

Haarigkeit ausserhalb (oberhalb) der log. Verteilung (innerhalb der hellgrauen Fläche)

Grundhaarigkeit

Haarigkeits-Mittelwert

$S_{1,5}$-Wert (=Summe [Haarlängenklasse x Häufigkeit] ab der Klasse 1,5)

Summenwert. Wert (=Summe [Haarlängenklasse x Häufigkeit] ab der Klasse des Haarigkeits-Mittelwertes)

$S_{1,0}$-Wert (=Summe [Haarlängenklasse x Häufigkeit] ab der Klasse 1,0)

$S_{0,5}$-Wert (=Summe [Haarlängenklasse x Häufigkeit] ab der Klasse 0,5)

7